# FASCICULE DE BREVET EUROPEEN

(11) **EP 2 068 991 B1**
(45) Date de publication et mention de la délivrance du brevet: **24.03.2010**
(21) Numéro de dépôt: 07803859.3
(22) Date de dépôt: 09.07.2007
(51) Int. Cl.: A61M 16/04

(54) **Dispositif medical intra-buccal**
Intraorales Medizinprodukt
Intraoral medical device

(30) Priorité: 12.07.2006 FR 0606360
(43) Date de publication de la demande: 17.06.2009
(73) Titulaire: Bastid, Christophe, 13009 Marseille (FR); Bey, Patrick, Abidjan 01 (CI); Poirot, Christophe, 88420 Moyenmoutier (FR); Bau, Giorgio, 88210 Le Mont (FR)
(72) Inventeur: Bastid, Christophe, 13009 Marseille (FR); Bey, Patrick, Abidjan 01 (CI); Poirot, Christophe, 88420 Moyenmoutier (FR); Bau, Giorgio, 88210 Le Mont (FR)
(74) Mandataire: Roman, Alexis
(86) Numéro de dépôt international: PCT/FR2007/001165
(87) Numéro de publication internationale: WO 2008/006968

(56) Documents cités:
- US-A- 1 498 810
- US-A- 3 730 179
- US-A- 4 848 331
- US-A- 5 318 017
- US-A1- 2004 129 272

## Description

La présente invention a pour objet un dispositif médical intra-buccal.

Elle concerne le domaine technique général des instruments médicaux et chirurgicaux utilisés lors d'une anesthésie ou lors de la réanimation d'un patient et plus particulièrement ceux utilisés lors d'interventions endoscopiques par voie haute.

Lors d'une endoscopie réalisée par voie haute, il est souvent nécessaire d'avoir recours à un apport d'oxygène qui s'effectue habituellement au moyen d'un conduit inséré dans la cavité buccale ou dans les narines ou à proximité immédiate de cefles-ci.

On connaît des dispositifs médicaux intra-buccaux constitués d'un ouvre-bouche équipé d'un conduit adapté pour injecter de l'oxygène dans la cavité buccale du patient. De tels dispositifs sont par exemple connus des documents US 4.495.945 (KENNETH), GB 2.173.105 (BAGNES) ou WO 91/08012 (BORODY). Cependant, ces dispositifs présentent l'inconvénient qu'une fraction importante de l'oxygène apporté se perd inutilement pour le patient et dangereusement pour les opérateurs (en particulier si un gaz anesthésiant est ajouté) dans la salle d'intervention.

L'oxygène doit donc être idéalement apporté le plus près possible des cordes vocales. On connaît ainsi des dispositifs médicaux intra-buccaux constitués d'un abaisse-langue comportant un canal d'injection d'oxygène débouchant au niveau du carrefour oro-pharyngé.

De tels dispositifs sont par exemple décrits dans les documents US 2.127.215 (GWATHMEY), US 2.705.959 (CAROL et al), US 3.756.244 (KINNEAR et al), US 4.881.542 (SCHMIDT et al), GB 1.558.171 (BURCHELL), DE 201.03.524.U (GREBKOWSKI), WO 80/00538 (LUOMANEN), WO 02/092144 (ELISHA MED. TECH. LTD).

Toutefois, la mise en place de tels dispositifs est impressionnante et souvent douloureuse pour les patients. Ils sont donc généralement introduits une fois que les patients sont anesthésiés et inconscients. Mais dans ce cas, les patients ne sont pas pré-oxygénés avant l'anesthésie, ce qui a pour conséquence d'augmenter les risques d'hypoxémie.

On connaît également des dispositifs médicaux intra-buccaux constitués d'un ouvre-bouche adapté pour recevoir un abaisse-langue comportant un canal d'injection d'oxygène débouchant au niveau du carrefour oro-pharyngé. De tels dispositifs sont par exemple décrits dans les documents US 4.270.531 (BLACHLY et al) ou US 2005/0217678 (McCORMICK et al). Toutefois, de tels dispositifs ne sont pas adaptés pour le passage d'instruments chirurgicaux et ne permettent pas une pré-oxygénation avant anesthésie. En outre, le dispositif décrit dans US 2005/0217678 (McCORMICK et al) ne permet pas d'insérer l'abaisse-langue dans l'ouvre-bouche sans déconnecter le système d'oxygénation de ce dernier.

Le dispositif le plus proche de l'invention est décrit dans le document US 4.848.331 (NORTHWAY-MEYER). Ce document divulgue un dispositif médical intra-buccal comportant :
- un ouvre-bouche formé d'un élément tubulaire arrangé pour définir un accès à la cavité buccale du patient,
- un abaisse-langue adapté pour s'insérer de manière amovible dans l'élément tubulaire lorsque ce dernier est positionné au niveau de la bouche du patient, ledit abaisse-langue comportant
   - un canal d'injection d'un gaz respirable débouchant au niveau de l'extrémité distale dudit abaisse-langue,
   - un conduit pour le passage d'instruments chirurgicaux débouchant à l'extrémité distale dudit abaisse-langue.

Un tel dispositif permet de pré-oxygéner le patient au début de l'intervention, puis d'insérer l'abaisse-langue dans l'élément tubulaire une fois l'anesthésie effective pour injecter l'oxygène au niveau du carrefour oro-pharyngé.

Toutefois, ce dispositif n'est pas totalement satisfaisant. En effet, lors des opérations chirurgicales, il est nécessaire de connecter la source d'oxygène sur un orifice spécifique disposé sur l'abaisse-langue. Il est donc indispensable d'avoir un autre orifice spécifique disposé sur le masque respiratoire lors de la phase de pré-oxygénation. Ce dispositif est en fait relativement complexe et difficile à manipuler.

Face à tous les inconvénients de l'art antérieur, le problème technique principal qu'envisage de résoudre l'invention est de permettre d'une manière particulièrement simple de pré-oxygéner un patient avant anesthésie puis d'oxygéner ce dernier en amenant l'oxygène au niveau du carrefour oro-pharyngé, sans introduction de matériel supplémentaire.

L'invention a également pour objectif de proposer un dispositif intra-buccal apte à aspirer les secrétions buccales ou autres, sans introduction de matériel supplémentaire.

Un autre objectif de l'invention est de proposer un dispositif médical intra-buccal du type décrit dans US 4.848.331 (NORTHWAY-MEYER) moins complexe et pouvant être manipulé plus facilement.

La solution proposée par l'invention est un dispositif médical intra-buccal comportant :
- un ouvre-bouche formé d'un élément tubulaire arrangé pour définir un accès à la cavité buccale du patient,
- un abaisse-langue adapté pour s'insérer de manière amovible dans l'élément tubulaire et comportant :
   - un canal d'injection d'un gaz respirable débouchant au niveau de l'extrémité distale dudit abaisse-langue,
   - un conduit pour le passage d'instruments chirurgicaux débouchant à l'extrémité distale dudit abaisse-langue,
et dans lequel l'ouvre-bouche comporte un conduit d'injection adapté pour être relié à une source de gaz respirable et configuré pour injecter ledit gaz dans la cavité buccale du patient lorsque l'abaisse-langue n'est pas inséré dans l'élément tubulaire et pour injecter ledit gaz dans le canal d'injection dudit abaisse-langue lorsque ce dernier est inséré dans ledit élément tubulaire. Il est ainsi possible d'amener le gaz respirable au niveau du carrefour oro-pharyngé sans qu'il soit nécessaire de connecter l'abaisse-langue à la source de gaz et sans avoir à déconnecter cette dernière de l'ouvre-bouche.

Selon une caractéristique avantageuse simplifiant la conception du dispositif objet de l'invention, le canal d'injection comporte un orifice d'entrée se positionnant en vis-à-vis de l'orifice de sortie du conduit d'injection lorsque l'abaisse-langue est inséré dans l'élément tubulaire.

Selon une autre caractéristique avantageuse de l'invention optimisant la circulation du gaz respirable, la paroi intérieure du canal d'injection située au niveau de l'orifice d'entrée et la paroi intérieure du conduit d'injection située au niveau de l'orifice de sortie sont biseautées selon une même direction.

Selon encore une autre caractéristique avantageuse de l'invention permettant d'injection efficacement le gaz respirable durant la phase de pré-oxygénation, le conduit d'injection s'étend vers l'arrière de l'élément tubulaire de manière à ce que l'extrémité distale sur laquelle est agencée l'orifice de sortie soit disposée vers le fond de la cavité buccale lorsque l'ouvre-bouche est mis en place.

Selon une autre caractéristique avantageuse de l'invention simplifiant la conception et permettant de laisser l'accès libre à la cavité buccale du patinent, le conduit d'injection est intégré à l'élément tubulaire et disposé latéralement le long d'un des bords inférieurs dudit élément tubulaire.

Selon une autre caractéristique avantageuse de l'invention permettant de limiter les pertes de gaz lors de la phase de pré-oxygénation, l'orifice de sortie du conduit d'injection est configuré de manière à ce que le gaz respirable soit éjecté vers le fond de la cavité buccale du patient. Et préférentiellement, l'orifice de sortie est disposé sur la partie supérieure du conduit d'injection, la paroi intérieure de ce dernier étant inclinée vers l'arrière au niveau dudit orifice de sortie.

Selon encore une autre caractéristique avantageuse destinée à préserver le confort du patient lors de la mise en place du dispositif objet de l'invention, "extrémité distale du conduit d'injection est biseautée.

Selon une autre caractéristique avantageuse de l'invention simplifiant la conception et permettant de laisser libre le passage des instruments chirurgicaux, le canal d'injection est intégré dans l'abaisse-langue et disposé latéralement le long d'un des bords intérieurs du conduit pour le passage desdits instruments chirurgicaux.

Selon encore une autre caractéristique avantageuse de l'invention, l'ouvre-bouche comporte un conduit d'aspiration des sécrétions.

Selon encore une autre caractéristique avantageuse de l'invention simplifiant la conception et permettant de laisser l'accès libre à la cavité buccale du patient, le conduit d'aspiration des sécrétions est intégré à l'élément tubulaire et disposé latéralement le long d'un des bords inférieurs dudit élément tubulaire.

Selon encore une autre caractéristique avantageuse facilitant la manipulation du dispositif objet de l'invention, le conduit d'aspiration comporte un orifice d'entrée situé sur la face externe de l'ouvre-bouche et sur lequel est agencé un embout destiné à être raccordé à un dispositif d'aspiration.

Selon encore une autre caractéristique avantageuse de l'invention permettant d'aspirer efficacement les secrétions, le conduit d'aspiration des secrétions s'étend vers l'arrière de l'élément tubulaire de manière à ce que sont extrémité distale sur laquelle est agencée un orifice d'aspiration soit disposée vers le fond de la cavité buccale lorsque l'ouvre-bouche est disposé au niveau de la bouche dudit patient.

Selon encore une autre caractéristique avantageuse de l'invention destinée à assurer une aspiration efficace des sécrétions, l'extrémité distale du conduit d'aspiration est biseauté, l'orifice d'aspiration étant agencé sur la face biseautée de ladite extrémité distale de façon à ce qu'il soit dirigé vers le bas.

Selon encore une autre caractéristique avantageuse de l'invention permettant d'aspirer les secrétions accumulées à l'entrée de la cavité buccale, le conduit d'aspiration comporte un orifice d'aspiration agencé sur une paroi de l'élément tubulaire. Et pour éviter que cet orifice soit obturé lors de la mise en place de l'abaisse-langue, ce dernier comporte une ouverture se positionnant en vis-à-vis de l'orifice d'aspiration lorsque ledit abaisse-langue est inséré dans l'élément tubulaire.

Selon encore une autre caractéristique avantageuse de l'invention, comporte un orifice d'aspiration des secrétions.

Selon encore une autre caractéristique avantageuse de l'invention permettant d'aspirer les secrétions au niveau de l'abaisse-langue sans avoir à relier ce dernier à un dispositif d'aspiration, l'orifice d'entrée du conduit d'aspiration de l'abaisse-langue se positionne en vis-à-vis d'une ouverture agencée sur le conduit d'aspiration de l'ouvre-bouche lorsque ledit abaisse-langue est inséré dans l'élément tubulaire.

Selon une autre caractéristique de l'invention permettant de faciliter l'extraction de l'abaisse-langue, ce dernier comporte deux ailettes disposées de part et d'autre de sa face externe, lesdites ailettes étant configurées pour s'agencer au niveau de deux ouvertures disposées de part et d'autre de la face externe de l'ouvre-bouche.

D'autres avantages et caractéristiques de l'invention apparaîtront mieux à la lecture de la description d'un mode de réalisation préférée qui va suivre, en référence aux dessins annexés, réalisés à titre d'exemples indicatifs et non limitatifs et sur lesquels :
- la figure 1 a est une vue schématique de face de l'ouvre-bouche,
- la figure 1b est une vue schématique de haut de l'ouvre-bouche de la figure 1 a,
- la figure 1c est une vue en coupe selon A-A de l'ouvre-bouche de la figure 1b montrant schématiquement l'agencement du conduit d'aspiration des sécrétions,
- la figure 1d est une vue en coupe selon B-B de l'ouvre-bouche de la figure 1 b montrant schématiquement l'agencement du conduit d'injection de gaz respirable,
- la figure 2a est une vue schématique de face de l'abaisse-langue,
- la figure 2b est une vue schématique de haut de l'abaisse-langue de la figure 2a,
- la figure 2c est une vue en coupe selon C-C de l'abaisse-langue de la figure 2b,
- la figure 2d est une vue en coupe selon D-D de l'abaisse-langue de la figure 2b montrant schématiquement l'agencement du canal d'injection du gaz respirable,
- la figure 3a est une vue schématique en perspective du dispositif intra-buccal conforme à l'invention, l'abaisse-langue étant inséré dans l'ouvre-bouche,
- la figure 3b est une vue en coupe verticale du dispositif intra-buccal de la figure 3a montrant la liaison entre le conduit d'injection de l'ouvre-bouche et le canal d'injection de l'abaisse-langue,
- la figure 3c est une vue en coupe verticale du dispositif intra-buccal de la figure 3a montrant la liaison entre le conduit d'aspiration des sécrétions de l'ouvre-bouche et l'abaisse-langue.

Le dispositif objet de l'invention est constitué d'un repousse langue 1 à usage unique destiné à être monté sur un ouvre-bouche 2 médical pour endoscopie par voie haute.

L'ouvre-bouche 2 est d'abord positionné au niveau de la bouche du patient lorsque ce dernier est conscient. Une fois que le patient est anesthésié et inconscient, l'abaisse-langue 1 est inséré dans l'ouvre-bòuche 2.

L'ouvre-bouche 2 est réalisé dans un matériau adapté à un usage médical. En pratique, il est obtenu par moulage en polymère thermoplastique à base de polyéthylène, d'autres matériaux et procédés de fabrication similaires pouvant être employés.

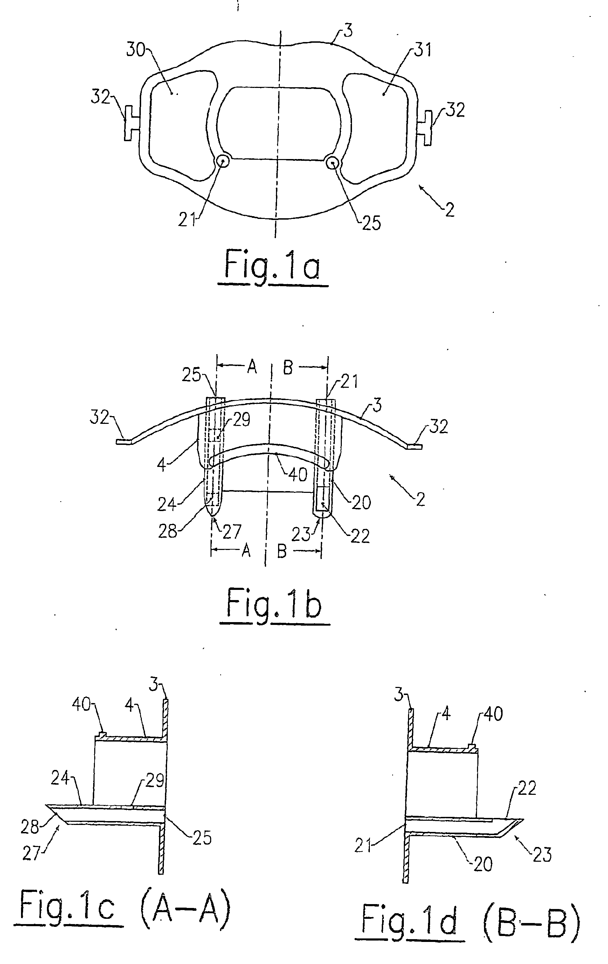

En se rapportant aux figures 1a, 1b, 1 c et 1 d, l'ouvre-bouche 2 comporte un élément tubulaire 4 s'insérant entre les dents du patient de manière à empêcher la bouche de se refermer.

Dans une variante de réalisation non représentée, l'ouvre-bouche 2 peut également être du type décrit dans US 4.848.331 (NORTHWAY-MEYER).

L'élément tubulaire 4 est arrangé pour définir un accès à la cavité buccale du patient, le médecin ou le chirurgien pouvant manipuler des instruments chirurgicaux au travers dudit élément tubulaire.

En pratique, l'élément tubulaire 4 est de forme générale rectangulaire légèrement incurvée au niveau de la partie distale de façon à s'adapter à la morphologie de la bouche. Les dimensions de l'élément tubulaire 4 pourront varier en fonction de la taille de la bouche du patient de manière à ce que sa mise en place soit la plus confortable possible.

L'élément tubulaire 4 comporte avantageusement des moyens pour maintenir en position l'ouvre-bouche 2 dans la bouche du patient lors de l'intervention.

En se rapportant au mode préféré de réalisation représentée sur les figures 1b, 1c et 1 d, l'élément tubulaire 4 comporte une partie en saillie 40 destinée à se placer derrière les dents de sorte que l'ouvre-bouche 2 ne puisse plus sortir de la bouche du patient une fois mis en place.

On peut également prévoir des rainures dans lesquelles viennent se caler les dents ou employer un revêtement relativement élastique dans lequel viennent s'enfoncer les dents.

L'ouvre-bouche 2 comporte avantageusement une partie externe 3 se plaquant contre le visage du patient.

En se rapportant aux figures annexées, cette partie externe 3 est agencée au niveau de la partie proximale de l'élément tubulaire 2. Deux ouvertures 30 et 31 sont préférentiellement disposées de part et d'autre de la face externe de l'ouvre-bouche 2.

De chaque côté de la partie externe 3 sont disposés des éléments 32 en forme de « T » (figure 1 a) destinés à recevoir un bandeau élastique s'étendant autour de la tête du patient de manière à maintenir l'ouvre-bouche 2 en position.

En se référant aux figures 1 a, 1 b, 1 c et 1 d, l'ouvre-bouche 2 comporte un conduit d'injection 20 adapté pour être relié à une source de gaz respirable et configuré pour injecter ledit gaz dans la cavité buccale du patient lorsque l'abaisse-langue 1 n'est pas inséré dans l'élément tubulaire 4. La présence d'un tel conduit d'injection permet de pré-oxygéner le patient d'une façon simple et particulièrement efficace avant qu'il ne soit anesthésié.

Selon le mode préféré de réalisation représentée sur les figures annexées, le conduit d'injection 20 est intégré à l'élément tubulaire 4 lors du moulage et disposé latéralement le long d'un de ses bords inférieurs de façon à laisser libre l'accès à la cavité buccale et dégager le passage des instruments chirurgicaux. Toutefois, il pourra être réalisé en utilisant toute technique permettant d'obtenir un résultat équivalent, par exemple en utilisant un tube séparé monté après moulage de l'ouvre-bouche.

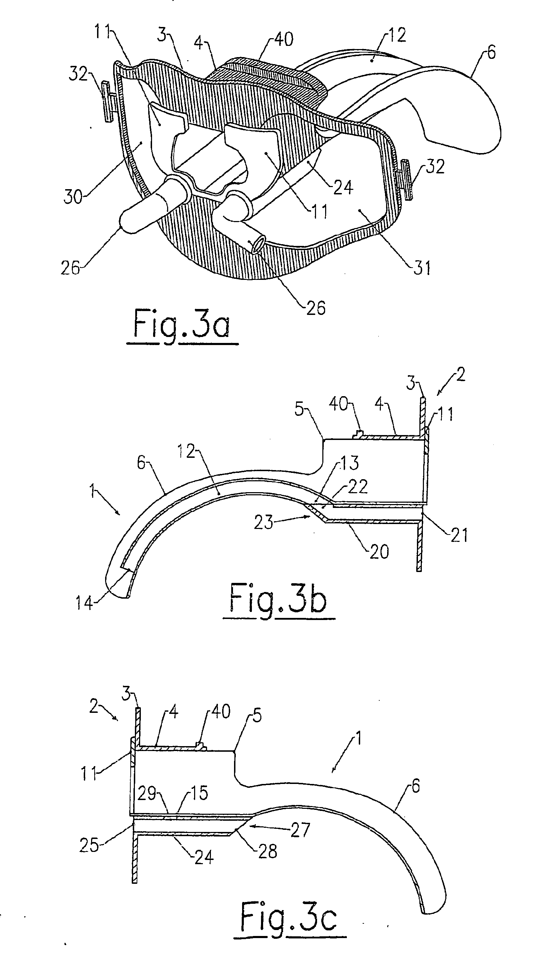

Le conduit d'injection 20 comporte un orifice d'entrée 21 situé sur la face externe de l'ouvre-bouche 2 et sur lequel peut être est agencé un embout 26 (figure 3a), amovible ou non, permettant de le raccorder facilement à une source d'oxygène ou de tout autres gaz respirables.

En se rapportant plus particulièrement aux figures 1b et 1d, le conduit d'injection 20 s'étend vers l'arrière de l'élément tubulaire 4 de manière à ce que son extrémité distale 23 sur laquelle est agencée l'orifice de sortie 22, soit disposée vers le fond de la cavité buccale lorsque l'ouvre-bouche 2 est mis en position dans la bouche du patient. Le gaz respirable est ainsi injecté relativement loin de l'entrée de la cavité buccale du patient ce qui évite des déperditions inutiles.

Conformément à la figure 1 d, l'extrémité distale 23 du conduit d'injection 20 est avantageusement biseautée pour ne pas gêner ou blesser le patient lorsque l'ouvre-bouche 2 est introduit dans la cavité buccale.

L'orifice de sortie 22 est agencé sur l'extrémité distale 23 et configuré de manière à ce que le gaz respirable soit éjecté vers le fond de la cavité buccale pour éviter toute déperdition inutile. En pratique, l'orifice de sortie 22 est disposé sur la partie supérieure du conduit d'injection 20 et la paroi intérieure de ce dernier est inclinée vers l'arrière au niveau dudit orifice de sortie.

En se référant aux figures 1 a, 1b, 1c et 1 d, l'ouvre-bouche 2 comporte préférentiellement un conduit d'aspiration des secrétions 24 destiné à évacuer les différentes secrétions (salive, sang, ...) présents dans la cavité buccale du patient et susceptibles de perturber le déroulement de l'intervention chirurgicale.

Selon un mode préféré de réalisation, le conduit d'aspiration 24 est intégré lors du moulage à l'élément tubulaire 4. Il est disposé latéralement le long d'un bord inférieur de l'élément tubulaire 4, avantageusement en vis-à-vis du conduit d'injection 20, de façon à laisser libre l'accès à la cavité buccale et dégager le passage des instruments chirurgicaux. Toutefois, il pourra être réalisé en utilisant toute technique permettant d'obtenir un résultat équivalent, par exemple en utilisant un tube séparé monté après le moulage de l'ouvre bouche 2.

Le conduit d'aspiration 24 comporte un orifice d'entrée 25 situé sur la face externe de l'ouvre-bouche 2 et sur lequel peut être agencé un embout 26 (figure 3a), amovible ou non, permettant de le raccorder facilement à un dispositif d'aspiration de type pompe.

En se rapportant plus particulièrement aux figures 1b et 1c, le conduit d'aspiration 24 s'étend vers l'arrière de l'élément tubulaire 4 de manière à ce que l'extrémité distale 27 sur laquelle est agencée l'orifice d'aspiration 28, soit disposée vers le fond de la cavité buccale lorsque l'ouvre-bouche 2 est mis en place. Cette configuration assure une aspiration efficace des sécrétions.

Conformément à la figure 1 c, l'extrémité distale 27 du conduit d'aspiration 24 est avantageusement biseautée de façon à ne pas gêner ou blesser le patient lorsque l'ouvre-bouche 2 est mis en place. D'une manière avantageuse, l'orifice d'aspiration 28 est agencé sur la face biseautée de l'extrémité distale 27 de façon à ce qu'il soit dirigé vers le bas pour rendre plus efficace l'aspiration.

En se référant aux figures 1a, 1b et 1c, le conduit d'aspiration 24 comporte un autre orifice d'aspiration 29 agencé sur une paroi de l'élément tubulaire 4. Cet orifice est destiné à aspirer les sécrétions accumulées au niveau de l'entrée de la cavité buccale et ne pouvant être aspirées par l'orifice 28.

L'abaisse-langue 1 est réalisé dans un matériau adapté à un usage médical. Il est avantageusement réalisé par moulage en polymère thermoplastique à base de polyéthylène, d'autres matériaux et procédés de fabrication similaires pouvant être employés.

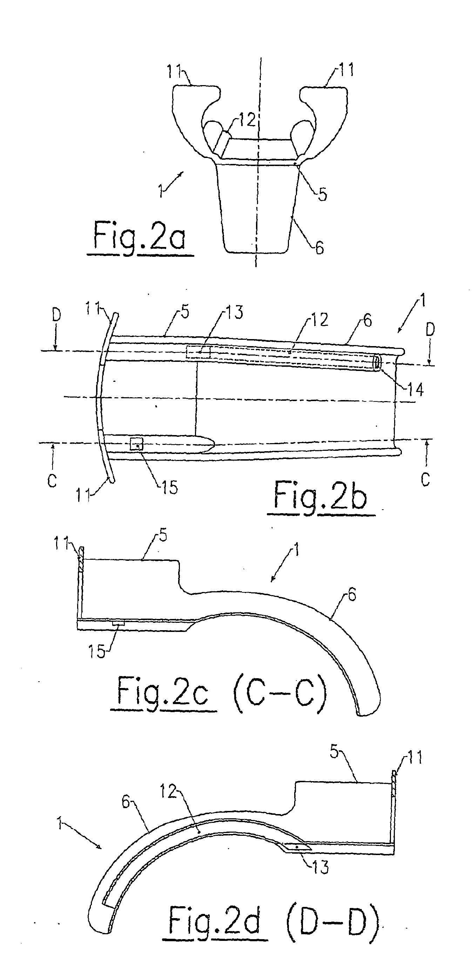

En se rapportant aux figures 2a, 2b, 2c et 2d, l'abaisse-langue 1 est formé :
- d'une embase 5 configurée pour s'emboîter de manière amovible dans l'élément tubulaire 4 de l'ouvre-bouche 2,
- et d'une canule 6 apte à pénétrer dans la bouche jusqu'au carrefour oro-pharyngé.

En pratique, l'embase 5 a une forme complémentaire de l'élément tubulaire 4. La canule 6 a une forme recourbée adaptée pour servir de cale-langue et éviter le basculement postérieur de la langue. On provoque ainsi le refoulement de la langue en bas et en avant afin d'assurer la liberté des voies aériennes supérieures.

Le retrait de l'abaisse-langue 1 est facilité par deux ailettes 11 d'extraction disposées de part et d'autre de la face externe dudit abaisse-langue. Pour faciliter l'extraction de l'abaisse-langue 1 et la préhension des deux ailettes 11, ces dernières sont configurées pour s'agencer au niveau des ouvertures 30 et 31 de la partie externe 3 de l'ouvre-bouche 2 (figure 3a).

La section transversale de l'abaisse-langue 1 est préférentiellement en forme de "U" ouvert vers le haut sur toute sa longueur de manière à former un chenal depuis l'extérieur de la cavité buccale vers le carrefour oro-pharyngé et permettant le passage d'endoscopes ou de tout autres instruments chirurgicaux, tout en assurant leur guidage. Dans une variante de réalisation non représentée, l'abaisse-langue 1 a une section transversale tubulaire sur toute sa longueur.

En se rapportant aux figures 2a, 2b et 2d, l'abaisse-langue 1 comporte un canal d'injection 12 d'un gaz respirable débouchant au niveau de l'extrémité distale dudit abaisse-langue.

Le canal d'injection 12 comporte un orifice de sortie 14 disposé au niveau du carrefour oro-pharyngé lorsque l'abaisse-langue 1 est inséré dans l'élément tubulaire 4 et que ce dernier est disposé dans la bouche du patient.

Selon un mode préféré de réalisation, le canal d'injection 12 est intégré à l'abaisse-langue 1 lors du moulage dans le conduit pour le passage d'instruments chirurgicaux. Il est avantageusement moulé latéralement le long d'un des bords intérieurs de ce conduit de façon à laisser libre l'accès à la cavité buccale et dégager le passage des instruments à introduire par la bouche du patient. Toutefois, il pourra être réalisé en utilisant toute technique permettant d'obtenir un résultat équivalent, par exemple en utilisant un tube séparé monté après le moulage de l'abaisse-langue 1.

Conformément aux figures 2d et 3b, le canal d'injection 12 comporte un orifice d'entrée 13 se positionnant en vis-à-vis de l'orifice de sortie 22 du conduit d'injection 20 lorsqu'il est inséré dans l'élément tubulaire 4. De cette manière, il n'est pas nécessaire de déconnecter le raccord 26 de l'ouvre-bouche 2 pour le raccorder à l'orifice d'entrée 13 du canal d'injection 12. En effet, le fait d'insérer l'abaisse-langue 1 dans l'élément tubulaire 4 a pour conséquence de diriger le gaz respirable dans le canal d'injection 12. D'autres dispositions permettant d'alimenter le canal d'injection 12 de manière équivalente peuvent évidemment être employées.

Dans le but de simplifier la conception du dispositif objet de l'invention, l'orifice de sortie 13 est agencé au niveau de l'embase 5.

Pour éviter un changement de direction brusque du flux de gaz respirable lors de l'entrée dans le canal d'injection 12, la paroi intérieure dudit canal située au niveau de l'orifice d'entrée 13 et la paroi intérieure du conduit d'injection 20 située au niveau de l'orifice de sortie 22 sont biseautées selon une même direction (figure 3b).

En se rapportant aux figures annexées, le conduit d'injection 20 comporte une partie en saillie dans la partie interne de l'élément tubulaire 4 et l'abaisse-langue 1 comporte une partie évidée ajustée à la forme de ladite partie en saillie. L'orifice de sortie 22 du conduit d'injection 20 est avantageusement agencé sur la partie en saillie dudit conduit d'injection et l'orifice d'entrée 13 du canal d'injection 12 est avantageusement agencé sur la partie évidée de l'abaisse-langue 1.

La partie en saillie et la partie évidée sont de forme complémentaire et sont ajustées de manière à assurer une bonne étanchéité au niveau de la connexion entre l'orifice d'entrée 13 et l'orifice de sortie 22. On peut éventuellement prévoir un moyen d'étanchéité de type joint au niveau de l'orifice de sortie 22 et/ou de l'orifice d'entrée 13.

Le fait d'avoir un abaisse-langue 1 avec une partie évidée apte à translater sur une partie en saillie disposée dans l'élément tubulaire 4 permet en outre d'assurer un guidage efficace dudit abaisse-langue lors de sa mise en place.

En se référant aux figures 2a, 2b et 2c, l'abaisse-langue 1 comporte une ouverture 15 se positionnant en vis-à-vis de l'orifice d'aspiration 29 agencé à l'intérieur de l'élément tubulaire 4 lorsqu'il est inséré dans ledit élément tubulaire (figure 3c). L'ouverture 15 est disposée dans l'embase 5 de l'abaisse-langue 1. De cette manière, la mise en place de l'abaisse-langue 1 dans l'élément tubulaire 1 n'a pas pour conséquence d'obturer l'orifice d'aspiration 29. Dans tous les cas, l'agencement de l'orifice d'aspiration 28 sur la face biseautée de l'extrémité distale 27 du conduit d'aspiration 24 fait que cet orifice 28 ne risque pas d'être obturé par l'insertion de l'abaisse-langue 1.

En se rapportant aux figures annexées, le conduit d'aspiration des sécrétions 24 comporte une partie en saillie dans la partie interne de l'élément tubulaire 4 et l'abaisse-langue 1 comporte une partie évidée ajustée à la forme de ladite partie en saillie. L'orifice d'aspiration 29 est agencé sur la partie en saillie du conduit d'aspiration 24 et l'ouverture 15 est agencée sur la partie évidée de l'abaisse-langue 1.

La partie en saillie et la partie évidée sont de forme complémentaire et sont ajustées de manière à assurer une bonne étanchéité au niveau de la connexion entre l'orifice d'aspiration 29 et l'ouverture 15. On peut éventuellement prévoir un moyen d'étanchéité de type joint au niveau de l'orifice d'aspiration 29 et/ou de l'ouverture 15.

Dans une variante de réalisation non représentée, l'abaisse-langue 1 est équipé d'un conduit d'aspiration des sécrétions intégré lors du moulage dans le conduit pour le passage des instruments chirurgicaux, en vis-à-vis du canal d'injection 12. Ce conduit d'aspiration comporte avantageusement un orifice d'aspiration agencé pour aspirer les sécrétions accumulées plus au fond de la cavité buccale.

De la même manière que décrit précédemment, l'orifice d'entrée du conduit d'aspiration de l'abaisse-langue 1 se positionne avantageusement en vis-à-vis de l'ouverture 15 lorsque ledit abaisse-langue est inséré dans l'élément tubulaire 4. De cette manière, il n'est pas nécessaire de déconnecter le raccord 26 de l'ouvre-bouche 2 pour le raccorder à l'orifice d'entrée du conduit d'aspiration de l'abaisse-langue 1.

## Revendications

1. Dispositif médical intra-buccal comportant :
• un ouvre-bouche (2) formé d'un élément tubulaire (4) arrangé pour définir un accès à la cavité buccale du patient,
• un abaisse-langue (1) adapté pour s'insérer de manière amovible dans l'élément tubulaire (4) et comportant :
- un canal d'injection (12) d'un gaz respirable débouchant au niveau de l'extrémité distale dudit abaisse-langue,
- un conduit pour le passage d'instruments chirurgicaux débouchant à l'extrémité distale dudit abaisse-langue,
**se caractérisant par le fait que** l'ouvre-bouche (2) comporte un conduit d'injection (20) adapté pour être relié à une source de gaz respirable et configuré pour injecter ledit gaz dans la cavité buccale du patient lorsque l'abaisse-langue (1) n'est pas inséré dans l'élément tubulaire (4) et pour injecter ledit gaz dans le canal d'injection (12) dudit abaisse-langue lorsque ce dernier est inséré dans ledit élément tubulaire.

2. Dispositif selon la revendication 1, dans lequel le canal d'injection (12) comporte un orifice d'entrée (13) se positionnant en vis-à-vis de l'orifice de sortie (22) du conduit d'injection (20) lorsque l'abaisse-langue (1) est inséré dans l'élément tubulaire (4).

3. Dispositif selon la revendication 2, dans lequel la paroi intérieure du canal d'injection (12) située au niveau de l'orifice d'entrée (13) et la paroi intérieure du conduit d'injection (20) située au niveau de l'orifice de sortie (22) sont biseautées selon une même direction.

4. Dispositif selon l'une des revendications précédentes, dans lequel le conduit d'injection (20) est intégré à l'élément tubulaire (4) et disposé latéralement le long d'un des bords inférieurs dudit élément tubulaire.

5. Dispositif selon l'une des revendications précédentes, dans lequel le conduit d'injection (20) s'étend vers l'arrière de l'élément tubulaire (4) de manière à ce que l'extrémité distale (23) sur laquelle est agencée l'orifice de sortie (22) soit disposée vers le fond de la cavité buccale lorsque l'ouvre-bouche (2) est disposé au niveau de la bouche du patient.

6. Dispositif selon l'une des revendications précédentes, dans lequel l'orifice de sortie (22) du conduit d'injection (20) est configuré de manière à ce que le gaz respirable soit éjecté vers le fond de la cavité buccale lorsque l'ouvre-bouche (2) est disposé au niveau de la bouche du patient.

7. Dispositif selon la revendication 6, dans lequel l'orifice de sortie (22) est disposé sur la partie supérieure du conduit d'injection (20), la paroi intérieure de ce dernier étant inclinée vers l'arrière au niveau dudit orifice de sortie.

8. Dispositif selon l'une des revendications précédentes, dans lequel l'extrémité distale (23) du conduit d'injection (20) est biseautée.

9. Dispositif selon l'une des revendications précédentes, dans lequel le canal d'injection (12) est intégré dans l'abaisse-langue (1) et disposé latéralement le long d'un des bords intérieurs du conduit pour le passage des instruments chirurgicaux.

10. Dispositif selon l'une des revendications précédentes, dans lequel l'ouvre-bouche (2) comporte un conduit d'aspiration des secrétions (24).

11. Dispositif selon la revendication 10, dans lequel le conduit d'aspiration (24) est intégré à l'élément tubulaire (4) et disposé latéralement le long d'un des bords inférieurs dudit élément tubulaire.

12. Dispositif selon l'une des revendications 10 ou 11, dans lequel le conduit d'aspiration (24) comporte un orifice d'entrée (25) situé sur la face externe de l'ouvre-bouche (2) et sur lequel est agencé un embout (26) destiné à être raccordé à un dispositif d'aspiration.

13. Dispositif selon l'une des revendications 10 à 12, dans lequel le conduit d'aspiration (24) s'étend vers l'arrière de l'élément tubulaire (4) de manière à ce que son extrémité distale (27) sur laquelle est agencée un orifice d'aspiration (28), soit disposée vers le fond de la cavité buccale lorsque l'ouvre-bouche (2) est disposé au niveau de la bouche du patient.

14. Dispositif selon la revendication 13, dans lequel l'extrémité distale (27) du conduit d'aspiration (24) est biseautée, l'orifice d'aspiration (28) étant agencé sur la face biseautée de ladite extrémité distale de façon à ce qu'il soit dirigé vers le bas.

15. Dispositif selon l'une des revendications 10 à 14, dans lequel le conduit d'aspiration (24) comporte un orifice d'aspiration (29) agencé sur une paroi de l'élément tubulaire (4).

16. Dispositif selon la revendication 15, dans lequel l'abaisse-langue (1) comporte une ouverture (15) se positionnant en vis-à-vis de l'orifice d'aspiration (29) lorsque ledit abaisse-langue est inséré dans l'élément tubulaire (4).

17. Dispositif selon l'une des revendications précédentes, dans lequel l'abaisse-langue (1) est équipé d'un conduit d'aspiration des sécrétions.

18. Dispositif selon la revendication 17, dans lequel l'orifice d'entrée du conduit d'aspiration de l'abaisse-langue (1) se positionne en vis-à-vis d'une ouverture (15) agencée sur le conduit d'aspiration de l'ouvre-bouche (2) lorsque ledit abaisse-langue est inséré dans l'élément tubulaire (4).

19. Dispositif selon l'une des revendications précédentes, dans lequel l'abaisse-langue (1) comporte deux ailettes (11) disposées de part et d'autre de sa face externe, lesdites ailettes étant configurées pour s'agencer au niveau de deux ouvertures (30, 31) disposées de part et d'autre de la face externe de l'ouvre-bouche (2).

## Claims

1. Intra-buccal medical device comprising:
• a mouth gag (2) formed of a tubular component (4) arranged to form an access to a patient's oral cavity,
• a tongue depressor (1) adapted to fit removably in the tubular component (4) and comprising:
- a channel (12) for injecting a breathable gas emerging at the distal end of the said tongue depressor,
- a tube for passing surgical instruments emerging at the distal end of the said tongue depressor,
**characterized in that** mouth gag (2) consists of an injection pipe (20) adapted so that it can be connected to a source of breathable gas and configured to inject the aforementioned gas into the patient's oral cavity when tongue depressor (1) is not inserted in tubular component (4) and to inject the aforementioned gas into injection channel (12) of said tongue depressor when the latter is inserted into the aforementioned tubular component.

2. Device according to claim 1, in which injection channel (12) consists of entrance orifice (13) positioned opposite exit orifice (22) of injection pipe (20) when tongue depressor (1) is inserted into tubular element (4).

3. Device according to the claim 2, in which the inner wall of injection channel (12) located at entrance orifice (13) and the inner wall of injection tube (20) located at exit orifice (22) are chamfered in the same direction.

4. Device according to one of the above claims, in which injection pipe (20) is incorporated in tubular component (4) and laid out laterally along one of the lower edges of the said tubular component.

5. Device according to one of the preceding claims, in which injection pipe (20) extends towards the rear of tubular component (4) so that distal end (23) on which is arranged exit orifice (22) is arranged towards the bottom of the oral cavity when mouth gag (2) is placed at the patient's mouth.

6. Device according to one of the preceding claims, in which exit orifice (22) of injection pipe (20) is configured so that the breathable gas is ejected towards the bottom of the oral cavity when mouth gag (2) is placed at the patient's mouth.

7. Device according to claim 6, in which exit orifice (22) is arranged on the upper part of injection pipe (20), the inner wall of the latter being inclined towards the rear of the said exit orifice.

8. Device according to one of the preceding claims, in which distal end (23) of injection pipe (20) is chamfered.

9. Device according to one of the preceding claims, in which the injection channel (12) is incorporated in tongue depressor (1) and laid out laterally along one of the inner edges of the pipe to allow passage of surgical instruments.

10. Device according to one of the preceding claims, in which mouth gag (2) consists of a secretions suction pipe (24).

11. Device according to claim 10, in which suction pipe (24) is incorporated in tubular component (4) and arranged laterally along one of the lower edges of the said tubular component.

12. Device according to one of claims 10 or 11, in which suction pipe (24) comprises an entry orifice (25) located on the outer face of mouth gag (2) and on which an end-piece (26) is arranged for connection to a breathing device.

13. Device according to one of claims 10 to 12, in which suction pipe (24) extends towards the rear of tubular component (4) so that its distal end (27) on which is arranged suction orifice (28) is arranged towards the bottom of the oral cavity when mouth gag (2) is placed at the patient's mouth.

14. Device according to claim 13, in which distal end (27) of suction pipe (24) is chamfered, suction pipe orifice (28) being arranged on the chamfered face of the said distal end so that it is directed downwards.

15. Device according to one of claims 10 to 14, in which suction pipe (24) has a suction orifice (29) arranged on a wall of tubular component (4).

16. Device according to claim 15, in which tongue depressor (1) comprises an orifice (15) positioning opposite suction orifice (29) when said tongue depressor is inserted in tubular component (4).

17. Device according to one of the preceding claims, in which tongue depressor (1) is equipped with a secretion suction pipe.

18. Device according to claim 17, in which the entrance orifice of the suction pipe of tongue depressor (1) is positioned opposite an orifice (15) arranged on the suction pipe of mouth gag (2) when the said tongue depressor is inserted in the tubular component (4).

19. Device according to one of the preceding claims, in which tongue depressor (1) has two fins (11) arranged on both sides of its outer face, the aforementioned fins being configured so as to be arranged at two orifices (30, 31) positioned on both sides of the outer face of mouth gag (2).

## Patentansprüche

1. Medizinische Vorrichtung für die Mundhöhle bestehend aus:
• einem Mundöffner (2), der aus einem rohrförmigen Element (4) besteht, das so gestaltet ist, dass der Zugang zur Mundhöhle des Patienten genau definiert festgelegt wird,
• einem Zungenspatel (1) der so gestaltet ist, dass er in das rohrförmige Element (4) ein- und ausgeführt werden kann und folgende Elemente aufweist:
- einem Einleitungskanal (12) für ein atembares Gas, der an dem distalen Ende des Zungenspatels mündet,
- einem Führungskanal für chirurgische Instrumente, der am distalen Ende des Zungenspatels mündet,
**dadurch gekennzeichnet, dass** der Mundöffner (2) eine Injektionsleitung (20) besitzt, die an die Quelle eines atembaren Gases angeschlossen werden kann und so gestaltet ist, dass das Gas in die Mundhöhle des Patienten strömt, wenn der Zungenspatel (1) nicht in das rohrförmige Element (4) eingeführt ist, und dass das Gas in den Einleitungskanal (12) des Zungenspatels eingeleitet wird, wenn dieser in das rohrförmige Element eingeführt ist.

2. Vorrichtung gemäß Patentanspruch 1, bei welcher der Einleitungskanal (12) eine Eingangsöffnung (13) besitzt, die der Ausgangsöffnung (22) der Injektionsleitung (20) gegenüber positioniert ist, wenn der Zungenspatel (1) in das rohrförmige Element (4) eingeführt ist.

3. Vorrichtung gemäß Patentanspruch 2, bei welcher die Innenwand des Einleitungskanals (12) in Höhe der Eingangsöffnung (13) und die Innenwand der Injektionsleitung (20) in Höhe der Ausgangsöffnung (22) in gleicher Richtung angeschrägt sind.

4. Vorrichtung gemäß einem der vorstehenden Patentansprüche, bei welcher die Injektionsleitung (20) in das rohrförmige Element (4) integriert ist und seitlich an einem der unteren Ränder des rohrförmigen Elements entlang verläuft.

5. Vorrichtung gemäß einem der vorstehenden Patentansprüche, bei welcher die Injektionsleitung (20) bis hinter das rohrförmige Element (4) reicht, so dass das distale Ende (23), an dem die Ausgangsöffnung (22) ausgebildet ist, nach hinten in die Mundhöhle zeigend angeordnet ist, wenn der Mundöffner (2) in den Mund des Patienten eingesetzt wird.

6. Vorrichtung gemäß einem der vorstehenden Patentansprüche, bei welcher die Ausgangsöffnung (22) der Injektionsleitung (20) so angeordnet ist, dass das austretende atembare Gas in nach hinten in die Mundhöhle strömt, wenn der Mundöffner (2) in den Mund des Patienten eingesetzt ist.

7. Vorrichtung gemäß Patentanspruch 6, bei welcher die Ausgangsöffnung (22) im oberen Teil der Injektionsleitung (20) angeordnet ist und die Innenwand der letzteren im Bereich der Ausgangsöffnung nach hinten geneigt ist.

8. Vorrichtung gemäß einem der vorstehenden Patentansprüche, bei welcher das distale Ende (23) der Injektionsleitung (20) angeschrägt ist.

9. Vorrichtung gemäß einem der vorstehenden Patentansprüche, bei welcher die Injektionsleitung (12) in den Zungenspatel (1) integriert ist und seitlich an einem der inneren Ränder des Führungskanals für die chirurgischen Instrumente entlang verläuft.

10. Vorrichtung gemäß einem der vorstehenden Patentansprüche, bei welcher der Mundöffner (2) eine Absaugleitung für Sekrete (24) besitzt.

11. Vorrichtung gemäß Patentanspruch 10, bei welcher die Absaugleitung (24) in das rohrförmige Element (4) integriert ist und seitlich an einem der unteren Ränder des rohrförmigen Elements entlang verläuft.

12. Vorrichtung gemäß einem der Patentansprüche 10 oder 11, bei welcher die Absaugleitung (24) eine an der Außenseite des Mundöffners (2) gelegene Eintrittsöffnung (25) besitzt, auf der ein Endstück (26) zum Anschluss an eine Absaugvorrichtung angeordnet ist.

13. Vorrichtung gemäß einem der Patentansprüche 10 bis 12, bei welcher die Absaugleitung (24) bis hinter das rohrförmige Element (4) reicht, so dass das distale Ende (27), an dem die Absaugöffnung (28) angeordnet ist, nach hinten in die Mundhöhle zeigt, wenn der Mundöffner (2) in den Mund des Patienten eingesetzt wird.

14. Vorrichtung gemäß Patentanspruch 13, bei welcher das distale Ende (27) der Absaugleitung (24) schräg angeschnitten ist und die Absaugöffnung (28) derart auf der angeschrägten Seite des genannten distalen Endes so angeordnet ist, dass dieses nach unten zeigt.

15. Vorrichtung gemäß einem der Patentansprüche 10 bis 14, bei welcher die Absaugleitung (24) eine Absaugöffnung (29) besitzt, die an einer Wand des rohrförmigen Elements (4) angeordnet ist.

16. Vorrichtung gemäß Patentanspruch 15, bei welcher der Zungenspatel (1) eine Öffnung (15) besitzt, die der Absaugöffnung (29) gegenüber liegt, wenn der Zungenspatel in das rohrförmige Element (4) eingeführt wird.

17. Vorrichtung gemäß einem der vorstehenden Patentansprüche, bei welcher der Zungenspatel (1) mit einer Absaugleitung für Sekrete versehen ist.

18. Vorrichtung gemäß Patentanspruch 17, bei welcher die Eintrittsöffnung der Absaugleitung des Zungenspatels (1) einer in der Absaugleitung des Mundöffners (2) vorgesehenen Öffnung (15) gegenüber zu liegen kommt, wenn der Zungenspatel in das rohrförmige Element (4) eingeführt wird.

19. Vorrichtung gemäß einem der vorstehenden Patentansprüche, bei welcher der Zungenspatel (1) zwei Flügel (11) besitzt, je einen auf jeder Außenseite, die so ausgebildet sind, dass sie sich an die beiden Öffnungen (30, 31) auf jeder Außenseite des Mundöffners (2) anlegen.
